# EUROPEAN PATENT APPLICATION

(11) **EP 3 360 465 A1**
(43) Date of publication of application: **15.08.2018**
(21) Application number: 17155200.3
(22) Date of filing: 08.02.2017
(51) Int. Cl.: A61B 5/00, A61B 5/11

(54) **A SYSTEM AND METHOD FOR BREAST MONITORING**

(71) Applicant: Koninklijke Philips N.V., 5656 AE Eindhoven (NL)
(72) Inventor: MENA BENITO, Maria Estrella, 5656 AE Eindhoven (NL); HUIJBREGTS, Laurentia Johanna, 5656 AE Eindhoven (NL); BOURQUIN, Yannyk Parulian Julian, 5656 AE Eindhoven (NL)
(74) Representative: de Haan, Poul Erik

(57) **Abstract**

There is provided a system (100) and method for breast monitoring. The system comprises a garment (102) that is adapted to at least partially cover at least one breast. The garment comprises at least one physiological characteristic sensor (104) that is configured to acquire one or more physiological characteristics of at least one breast of a subject wearing the garment (102). The system (100) also comprises at least one motion sensor (106) that is configured to acquire information on the subject wearing the garment (102).

## Description

### Technical Field of the Invention

The invention relates to the field of monitoring a subject and, in particular, to a system and method for breast monitoring.

### Background to the Invention

It is important for women to undergo regular breast examinations. Usually, breast examinations need to be performed by the women themselves. Healthcare specialist recommend that women perform breast self-examination (BSE) regularly. By regular breast self-examination, women can become familiar with how their breasts normally look and feel. This can be helpful since women can then more readily detect any changes (such as lumps) that occur in the breasts. Since many women naturally have asymmetric differences between their right and left breasts, the key to a successful breast self-examination is to learn how to find changes in the breasts and to monitor to observe whether those changes persist over time.

Many breast problems are first discovered by women performing breast self-examination. For example, breast self-examination can be important to signal breast cancer in an early stage or to detect complications with breast implants (such as the implant rupturing or leaking silicone). Regular breast self-examination can also be useful in avoiding the development of mastitis in breastfeeding mothers, which can happen when a milk duct becomes blocked. It can also be useful for estimating a milk volume in the breasts of breastfeeding mothers, determining changes in the breasts after each milk extraction period and as a tool for assisting the mother in determining which breast should be used for the next extraction.

However, while most women are aware that regular breast self-examination is important, many women do not examine their breasts. This is often due to the women not being educated in how to perform a self-examination or due to the women experiencing difficulty in performing a self-examination or finding the experience frustrating or embarrassing. Also, even in the case of women that perform self-examination of their breasts, it can often be performed incorrectly and areas of concern can be missed, which can give the women a false sense of security.

Certain systems exist that can support a subject in breast monitoring. For example, CN 104223385 A discloses an intelligent bra for early detection of breast cancer. The intelligent bra carries computer micro-processing chips, which include a temperature sensor, a blood flow sensor and an oxyhemoglobin quantity sensor for the acquisition of breast tissue temperature, blood flow and hemoglobin amount. However, while these types of systems may provide an easier approach for breast self-examination, the measurements acquired by these systems can be prone to errors, particularly since the subject is likely to move during acquisition of the measurements. The errors that are introduced into the measurements acquired can result in important changes in the breast being missed or misinterpreted.

There is thus a need for an improved system and method for breast monitoring.

### Summary of the Invention

As noted above, the limitation with existing systems that support a subject in breast monitoring is that the measurements acquired can be prone to errors, which can result in important changes in the breast being missed or misinterpreted. It would thus be valuable to have an improved system and method for breast monitoring, which overcomes the existing problems.

Therefore, according to a first aspect of the invention, there is provided a system for breast monitoring. The system comprises a garment adapted to at least partially cover at least one breast, the garment comprising at least one physiological characteristic sensor configured to acquire one or more physiological characteristics of at least one breast of a subject wearing the garment. The system also comprises at least one motion sensor configured to acquire information on the subject wearing the garment.

In some embodiments, the information may comprise any one or more of information indicative of a posture of the subject, information indicative of an orientation of the subject, information indicative of a position of the subject, and information indicative of a movement of the subject.

In some embodiments, at least one motion sensor may be positioned over each breast of the subject. In some embodiments, the garment may comprise any one or more of: one or more cups, each cup adapted to at least partially cover a breast of the subject, wherein one or more motion sensors are positioned in at least one of the one or more cups or between the one or more cups, or in at least one of the one or more cups and between the one or more cups; and one or more straps, wherein one or more motion sensors are positioned in at least one of the one or more straps.

In some embodiments, the system may further comprise a processor configured to process the one or more acquired physiological characteristics based on the acquired information. In some embodiments, the garment may comprise the processor.

According to a second aspect of the invention, there is provided a method for breast monitoring. The method comprises controlling at least one physiological characteristic sensor of a garment adapted to at least partially cover at least one breast to acquire one or more physiological characteristics of at least one breast of a subject wearing the garment and controlling at least one motion sensor to acquire information on the subject wearing the garment.

In some embodiments, the method may further comprise processing the one or more acquired physiological characteristics based on the acquired information. In some embodiments, processing the one or more physiological characteristics may comprise any one or more of: comparing one or more physiological characteristics acquired when the information is indicative that the subject is in one posture with one or more corresponding physiological characteristics acquired when the information is indicative that the subject is in the same posture at another time; comparing one or more physiological characteristics acquired when the information is indicative that the subject is in one posture with one or more corresponding physiological characteristics acquired when the information is indicative that the subject is in another posture, taking into account a transfer function of the one or more characteristics between the two postures; comparing one or more physiological characteristics acquired when the information is indicative that the subject is in an active state with one or more corresponding physiological characteristic acquired when the information is indicative that the subject is in an inactive state, taking into account a transfer function of the one or more characteristics between the active and inactive state; comparing one or more physiological characteristics acquired during a defined motion, immediately following a defined motion, or both during and immediately following a defined motion with one more corresponding physiological characteristics acquired during the same defined motion, immediately following the same defined motion, or both during and immediately following the same defined motion; analysing one or more physiological characteristics acquired during a defined motion, immediately following a defined motion, or both during and immediately following a defined motion; and comparing one or more physiological characteristics acquired from at least one physiological characteristic sensor positioned over a first breast with one or more corresponding physiological characteristics acquired from at least one physiological characteristic sensor positioned over a second breast when the information is indicative of a change in the movement of the subject.

In some embodiments, processing the one or more physiological characteristics may comprise excluding from processing one or more physiological characteristics acquired when the information is indicative of the movement of the subject exceeding a threshold. In some embodiments, excluding one or more physiological characteristics may comprise ceasing to acquire the one or more physiological characteristics from the at least one physiological characteristic sensor. In some embodiments, the method may further comprise continuing to acquire and process the one or more physiological characteristics from the at least one physiological characteristic sensor after a predetermined time period has expired following the movement or when the information is indicative that the movement of the subject is equal to or below the threshold.

In some embodiments, the acquired one or more physiological characteristics may be processed to identify any one or more of an abnormality in the at least one breast of the subject and a milk volume in the at least one breast of the subject. In some embodiments, the method may further comprise controlling one or more user interfaces to output information indicative of any one or more of the abnormality and milk volume. According to a third aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to the aspects and embodiments described above, the limitations of existing techniques are addressed. In particular, according to the above-described aspects and embodiments, breast monitoring of a subject can be performed in a simple, unobtrusive and private manner through a smart sensing garment. Moreover, errors in acquired measurements can be reduced through use of the at least one motion sensor in combination with the smart sensing garment. In this way, the system can obtain more reliable information regarding any changes in the condition of the at least one breast.

There is thus provided an improved system and method for breast monitoring, which overcomes the existing problems.

### Brief Description of the Drawings

For a better understanding of the invention, and to show more clearly how it may be carried into effect, reference will now be made, by way of example only, to the accompanying drawings, in which:
Figure 1 is a block diagram of a system according to an embodiment; and
Figure 2 is a flow chart illustrating a method according to an embodiment.

### Detailed Description of the Preferred Embodiments

As noted above, the invention provides an improved system and method for breast monitoring, which overcomes the existing problems.

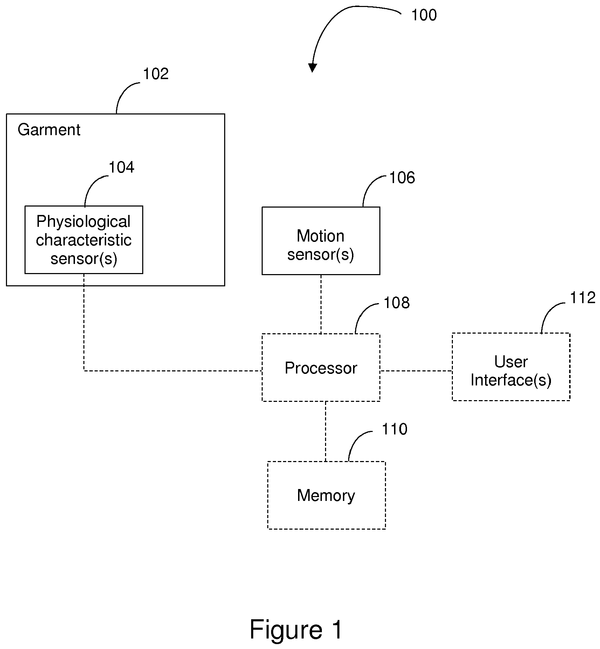

**Figure 1** illustrates an example of a system 100 that can be used for breast monitoring according to an embodiment.

With reference to Figure 1, the system 100 comprises a garment (or an item of clothing) 102 that is adapted to at least partially cover at least one breast of a subject. The garment 102 can be any garment (or item of clothing) that is adapted to at least partially cover at least one breast.

Examples of a garment 102 adapted to at least partially cover at least one breast include, but are not limited to, a brassiere (or bra), a bodice, a vest, a top (such as a t-shirt, tank top, crop top, tube top, bandeau, blouse, or any other top), or any other garment that is adapted to at least partially cover at least one breast. In some embodiments, the garment 102 adapted to at least partially cover at least one breast can comprise one or more cups, where each cup is adapted to at least partially cover a breast of the subject. Alternatively or in addition, the garment 102 adapted to at least partially cover at least one breast can comprise one or more straps adapted to hold the garment in place to at least partially cover the breast of the subject. Although examples have been provided for the form the garment 102 that is adapted to at least partially cover at least one breast may take, it will be understood that other forms for the garment 102 are also possible. The garment 102 can take any form suitable to at least partially cover at least one breast of a subject wearing the garment.

The garment 102 that is adapted to at least partially cover at least one breast comprises at least one physiological characteristic sensor 104 configured to acquire one or more physiological characteristics (or measurements) of at least one breast of a subject wearing the garment 102. Specifically, the at least one physiological characteristic sensor 104 is configured to acquire information on the condition of the at least one breast of a subject wearing the garment 102. In some embodiments, for example, at least one physiological characteristic sensor 104 may be integrated into the garment. In other embodiments, for example, at least one physiological characteristic sensor 104 may be provided with an attachment mechanism (such as Velcro) to allow the physiological characteristic sensor 104 to be removeably attached to the garment 102.

In some embodiments, at least one physiological characteristic sensor 104 may be positioned over each breast of the subject. In this way, it is possible to acquire objective information of the condition of both breasts independently. In an embodiment where the garment 102 comprises one or more cups, with each cup adapted to at least partially cover a breast of the subject, at least one physiological characteristic sensor 104 may be positioned in at least one of the one or more cups, or between the one or more cups, or in at least one of the one or more cups and between the one or more cups. In an embodiment where the garment 102 additionally or alternatively comprises one or more straps adapted to hold the garment 102 in place to at least partially cover the breast of the subject, at least one physiological characteristic sensor 104 may be positioned in at least one of the one or more straps. In some embodiments, the garment 102 may comprise one or more grids of physiological characteristic sensors 104.

As mentioned earlier, the at least one physiological characteristic sensor 104 is configured to acquire information on the condition of the at least one breast of a subject wearing the garment 102. Thus, the one or more physiological characteristics acquired by the at least one physiological characteristic sensor 104 can comprise information on the condition of the at least one breast. For example, the one or more physiological characteristics acquired by the at least one physiological characteristic sensor 104 can comprise characteristics related to the anatomy of the at least one breast. Examples of a physiological characteristic sensor 104 include, but are not limited to, a temperature sensor, a fluid (for example, blood) volume sensor, a stretch sensor, a tissue rigidity sensor, an internal body structure sensor, or any other sensor, or any combination of sensors, suitable to acquire one or more physiological characteristics of at least one breast of a subject wearing the garment 102.

A temperature sensor may be any type of sensor, or any combination of sensors, suitable to acquire a temperature measurement from at least one breast of a subject. The acquired temperature measurement is indicative of the condition of the at least one breast. In some embodiments, the temperature measurement may comprise one or more temperature readings or a heat pattern from at least one breast. Examples of a temperature sensor include, but are not limited to, a thermometer, a thermistor, a thermocouple, a fiber optic sensor, or any other temperature sensor, or any combination of temperature sensors.

A blood volume sensor may be any type of sensor, or any combination of sensors, suitable to acquire a blood volume measurement from at least one breast of a subject. The acquired blood volume measurement is indicative of the condition of the at least one breast. In some embodiments, the blood volume sensor may be configured to use different wavelengths (such as red and infrared) to illuminate the skin of at least one breast of a subject to measure a blood oxygenation level. Examples of a blood volume sensor include, but are not limited to, a photoplethysmogram (PPG) sensor, or any other blood volume sensor, or any combination of blood volume sensors.

A fluid volume sensor may be any type of sensor, or any combination of sensors, suitable to acquire a fluid volume measurement from at least one breast of a subject. The acquired fluid volume measurement is indicative of the condition of the at least one breast. In some embodiments, the fluid volume sensor may be configured to determine a fluid volume of at least one breast of a subject in general or, more specifically, to provide information on the emptiness of fullness of at least one breast of a nursing mother. Examples of a fluid volume sensor include, but are not limited to, an impedance sensor, or any other fluid volume sensor, or any combination of fluid volume sensors.

A strain sensor may be any type of sensor, or any combination of sensors, suitable to acquire a strain measurement from at least one breast of a subject. The acquired strain measurement is indicative of the condition of the at least one breast. In some embodiments, for example, the strain sensor can be configured to detect changes in stretching of the skin of at least one breast of a subject, swelling of at least one breast of a subject, or similar. Examples of a strain sensor include, but are not limited to, a strain gauge, a touch sensor (such as a grid of swept frequency capacitive sensors, which can detect a touch event and/or configurations of the body), or any other strain sensor, or any combination of strain sensors.

A tissue rigidity sensor may be any type of sensor, or any combination of sensors, suitable to acquire a tissue rigidity measurement from at least one breast of a subject. The acquired tissue rigidity measurement is indicative of the condition of the at least one breast. In some embodiments, for example, a tissue rigidity sensor may be configured to distinguish between hard and soft tissue in at least one breast of a subject or to provide information on anatomical changes in at least one breast of a subject such as breast shape, breast tissue stiffness, breast thickening, lumps, dimpling, or any other anatomical changes. Examples of a tissue rigidity sensor include, but are not limited to, a pressure sensor, an ultrasound elastrography sensor, or any other tissue rigidity sensor, or any combination of tissue rigidity sensors.

An internal body structure sensor may be any type of sensor, or any combination of sensors, suitable to acquire the internal body structure from at least one breast of a subject. The acquired internal body structure is indicative of the condition of the at least one breast. In some embodiments, for example, an internal body structure sensor may be configured to detect changes in an anatomical structure within the at least one breast (such as a change in size of mammary glands, a blocked milk duct, an apparition of a cyst, or any other change in an anatomical structure). Examples of an internal body structure sensor include, but are not limited to, an ultrasound sensor, or any other internal body structure sensor, or any combination of internal body structure sensors.

As shown in Figure 1, the system 100 also comprises at least one motion (or activity or inertial) sensor 106 configured to acquire information on the subject wearing the garment 102. A motion (or activity or inertial) sensor 106 may be any type of sensor, or any combination of sensors, suitable to acquire information on the subject wearing the garment 102. Examples of a motion sensor 106 include, but are not limited to, an accelerometer, a gyroscope, a magnetometer, or any other motion sensor, or any combination of motion sensors. The information acquired by the at least one motion sensor 106 can comprise any one or more of information indicative of a posture of the subject, information indicative of an orientation of the subject, information indicative of a position of the subject, information indicative of a movement or displacement of the subject (such as movement of the body or a part of the body of the subject, a breathing or respiration movement of the subject, or any other movement of the subject), or any other information, or any combination of information on the subject. In some embodiments, information indicative of a change in orientation of the subject can be used to determine a movement of the subject.

In the illustrated example embodiment of Figure 1, one or more of the at least one motion sensors 106 are external to (i.e. separate to or remote from) the garment 102 that is adapted to at least partially cover at least one breast of a subject. For example, in some embodiments, another garment may comprise one or more of the at least one motion sensors 106. The other garment can be any garment that is adapted to be worn by a subject. Examples of the other garment include, but are not limited to, underpants, or any other garment that is adapted to be worn by a subject.

Alternatively or in addition, in some embodiments, a mobile device (such as a mobile phone or any other mobile device) that can be carried by the subject (for example, in a pocket of a garment worn by the subject) may comprise one or more of the at least one motion sensors 106. Alternatively or in addition, in some embodiments, a wearable device may comprise one or more of the at least one motion sensors 106. A wearable device can be any device suitable to be worn on or around a part of the body of the subject, such as the neck of the subject, the wrist of the subject, the waist of the subject, the back of the subject, the chest of the subject, or any other part of the body of the subject. In a neck-worn embodiment, a wearable device may be in the form of a pendant that can be worn on a cord, chain, necklace, or collar around the neck of the subject. In a wrist-worn embodiment, a wearable device may be in the form of a wrist band, a wrist strap or a watch that can be worn on the wrist of the subject.

Although not illustrated in the example embodiment of Figure 1, alternatively or in addition to one or more of the at least one motion sensors 106 being external to (i.e. separate to or remote from) the garment 102 that is adapted to at least partially cover at least one breast of a subject, the garment 102 itself can comprise one or more of the at least one motion sensors 106. In some embodiments, at least one motion sensor 106 may be positioned over each breast of the subject. In an embodiment where the garment 102 comprises one or more cups, with each cup adapted to at least partially cover a breast of the subject, one or more motion sensors 106 may be positioned in at least one of the one or more cups (for example, such that the one or motion sensors 106 are in direct or indirect contact with at least one breast itself), or between the one or more cups, or in at least one of the one or more cups and between the one or more cups. In an embodiment where the garment 102 additionally or alternatively comprises one or more straps adapted to hold the garment 102 in place to at least partially cover the breast of the subject, one or more motion sensors 106 may be positioned in at least one of the one or more straps. In an embodiment where one or more motion sensors 106 are positioned between the one or more cups, those motion sensors 106 can be used to acquired information indicative of the overall posture of the subject. In some embodiments, one or more motion sensors 106 may be placed in close proximity to an arm of the subject such that information on the movement of the arm of the subject can be acquired.

Although examples have been provided for the type of the at least one physiological characteristic sensor 104 and the at least one motion sensor 106 and the arrangement of those sensors, it will be understood that other types of sensor, or combinations of sensors, and sensor arrangements are also possible.

As illustrated in the example embodiment of Figure 1, according to some embodiments, the system 100 may further comprise a processor 108 configured to process the one or more acquired physiological characteristics based on the acquired information. The processor 108 can control the operation of the system 100 and can implement the method described herein. The processor 108 can comprise one or more processors, processing units, multi-core processors or modules that are configured or programmed to control the system 100 in the manner described herein. In particular implementations, the processor 108 can comprise a plurality of software and/or hardware modules that are each configured to perform, or are for performing, individual or multiple steps of the method described herein.

In the illustrated example embodiment of Figure 1, the processor 108 is external to (i.e. separate to or remote from) the garment 102 that is adapted to at least partially cover at least one breast of a subject. For example, the cloud or a device that is external to the garment 102 may comprise the processor 108. The device can be a computer, a mobile device (for example, a phone such as a smartphone, a tablet, a laptop, a watch such as a smart watch, or any other mobile device), or any other device. Alternatively or in addition, the garment 102 that is adapted to at least partially cover at least one breast of a subject may itself comprise the processor 108 according to some embodiments.

As illustrated in the example embodiment of Figure 1, according to some embodiments, the system 100 may also comprise a memory 110 configured to store program code that can be executed by the processor 108 to perform the method described herein. In the illustrated example embodiment of Figure 1, the memory 110 is external to (i.e. separate to or remote from) the garment 102 that is adapted to at least partially cover at least one breast of a subject. For example, the cloud or a device that is external to the garment 102 may comprise the memory 110 (which may be the same device or a different device to that comprising the processor 108). The device can be a computer, a mobile device (for example, a phone such as a smartphone, a tablet, a laptop, a watch such as a smart watch, or any other mobile device), or any other device.

Alternatively or in addition, the garment 102 that is adapted to at least partially cover at least one breast of a subject may itself comprise the memory 110 according to some embodiments. For example, in an embodiment where the garment 102 comprises one or more straps adapted to hold the garment 102 in place to at least partially cover the breast of the subject, a strap (such as a strap adapted to be worn around the rib cage of the subject) can comprise the memory 110. In some embodiments, the memory 110 can be integrated in the garment. In other embodiments, the memory 110 may be provided with an attachment mechanism (such as Velcro) to allow the memory 110 to be removeably attached to the garment.

A memory 110 can be used to store information resulting from the method described herein. Thus, for example, a memory 110 may be used to store the one or more acquired physiological characteristics, the information acquired from the one or more motion sensors 106, any information resulting from the processing of the one or more acquired physiological characteristics, or any other information resulting from the method, or any combination of information. The processor 108 may be configured to control a memory 110 to store the information resulting from the method described herein.

As illustrated in the example embodiment of Figure 1, according to some embodiments, the system 100 may also comprise at least one user interface 112. A user interface 112 may be for use in providing a user of the system 100 with information resulting from the method according to the invention. A user can be any user of the system 100. For example, a user may be the subject themselves, a healthcare provider, a healthcare specialist, a caregiver, a family member, or any other user.

As illustrated in the example embodiment of Figure 1, according to some embodiments, one or more of the at least one user interfaces 112 can be external to (i.e. separate to or remote from) the garment 102 that is adapted to at least partially cover at least one breast of a subject. For example, a device that is external to the garment 102 may comprise one or more of the at least one user interfaces 112 (which may be the same device or a different device to that comprising the processor 108 and/or the memory 110). The device can be a computer, a mobile device (for example, a phone such as a smartphone, a tablet, a laptop, a watch such as a smart watch, or any other mobile device), or any other device. Alternatively or in addition, the garment 102 that is adapted to at least partially cover at least one breast of a subject may itself comprise one or more of the at least one interfaces 112 according to some embodiments.

The processor 108 of the system 100 may be configured to control one or more of the at least one user interfaces 112 to provide information resulting from the method described herein. For example, in some embodiments, the processor 108 may be configured to control one or more user interfaces 112 to render (or output or display) the one or more acquired physiological characteristics, the information acquired from the one or more motion sensors 106, any information resulting from the processing of the one or more acquired physiological characteristics, or any other information resulting from the method, or any combination of information. In some embodiments, a user interface 112 can be configured to render an alert the subject when there are abnormal changes detected in the at least one breast. In addition, in some embodiments, a user interface 112 can be configured to render an indication of one or more locations of abnormality. Where no abnormalities are found, a user interface 112 can optionally be configured to render an output indicating that no abnormalities are found. In some embodiments, a user interface 112 can optionally be configured to render an output instructing the subject to perform a manoeuvre (such as pressing on at least one breast, laying down, or any other manoeuvre).

Alternatively or in addition to providing information, a user interface 112 may be configured to receive a user input. In other words, a user interface 112 may allow the user of the system 100 to manually enter data, instructions, or information. The processor 108 may be configured to acquire the user input from one or more user interfaces 112.

Thus, a user interface 112 may be any user interface that enables rendering (or outputting or displaying) of information to a user of the system 100 and, alternatively or in addition, that enables a user of the system 100 to provide a user input, interact with and/or control the system 100. For example, a user interface 112 may comprise one or more switches, one or more buttons, a keypad, a keyboard, a mouse, a touch screen or an application (for example, on a smart device such as a tablet or smartphone), a display screen, a graphical user interface (GUI) or any other visual component, one or more speakers, one or more microphones or any other audio component, one or more lights, a component for providing tactile feedback (such as a vibration function), or any other user interface, or combination of user interfaces. In some embodiments, the user interface 112 that is controlled to render (or output or display) information to the user of the system 100 may be the same user interface as that which enables the user to provide a user input, interact with and/or control the system 100.

In an example embodiment where the garment 102 that is adapted to at least partially cover at least one breast of a subject comprises one or more of the at least one user interfaces 112, the one or more user interfaces 112 of the garment can, for example, comprise one or more light indicators to provide information resulting from the method described herein. In an example embodiment where one or more of the at least one user interfaces 112 are external to (i.e. separate to or remote from) the garment 102, the one or more external user interfaces 112 can, for example, comprise any one or more of one or more displays to render information resulting from the method in a visual format and one or more speakers to render information resulting from the method in an audio format.

Although not illustrated in the example embodiment of Figure 1, the system 100 may comprise one or more communications interfaces (or circuitry) for enabling the components, interfaces, units, memories, sensors and devices of the system 100 to communicate with each other, which may be wirelessly or via a wired connection. In some embodiments, for example, the garment 102 may comprise a communications interface (such as a transmitter) configured to transmit the one or more acquired physiological characteristics to the processor 108.

It will be appreciated that Figure 1 only shows the components required to illustrate an example embodiment of the invention and, in a practical implementation, the system 100 may comprise additional components to those shown. For example, although not illustrated in Figure 1, the system 100 may comprise one or more batteries or other power supplies for powering the components of the system 100 or means for connecting the components of the system 100 to a mains power supply.

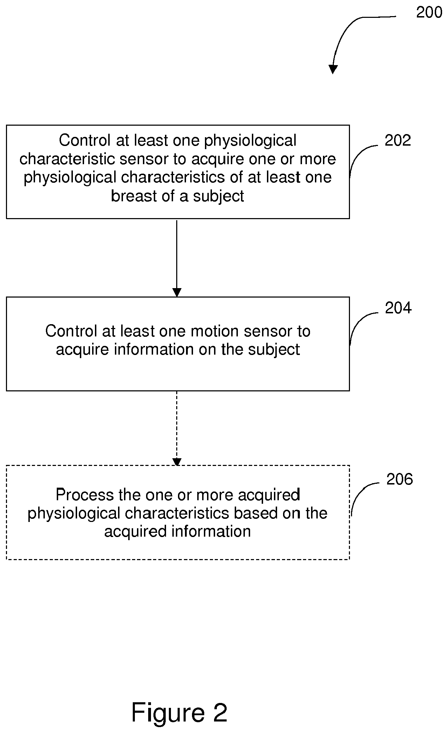

**Figure 2** illustrates a method 200 for breast monitoring according to an embodiment. The illustrated method 200 can generally be performed by or under the control of the processor 108 of the system 100.

With reference to Figure 2, at block 202, at least one physiological characteristic sensor 104 of a garment adapted to at least partially cover at least one breast is controlled to acquire one or more physiological characteristics (or measurements) of at least one breast of a subject wearing the garment. As mentioned earlier, the one or more physiological characteristics acquired by the at least one physiological characteristic sensor 104 can comprise information on the condition of the at least one breast. The processor 108 of the system 100 may control the at least one physiological characteristic sensor 104 of the garment 102 to acquire the one or more physiological characteristics. In some embodiments, a plurality of different physiological characteristics may be acquired from at least one breast of the subject simultaneously. In some embodiments, one or more physiological characteristics may be acquired from both breasts simultaneously.

At block 204, at least one motion sensor 106 is controlled to acquire information on the subject wearing the garment. As previously mentioned, the information acquired by the at least one motion sensor 106 can comprise any one or more of information indicative of a posture of the subject, information indicative of an orientation of the subject, information indicative of a position of the subject, information indicative of a movement or displacement of the subject (such as movement of the body or a part of the body of the subject, a breathing or respiration movement of the subject, or any other movement of the subject), or any other information, or any combination of information on the subject. The processor 108 of the system 100 may control the at least one motion sensor 106 to acquire the information on the subject. The acquired information allows the one or more acquired physiological characteristics to be correlated to the subject (for example, to the posture, orientation, positon, and/or movement of the subject). Thus, optionally at block 206, the one or more acquired physiological characteristics may be processed based on the acquired information according to some embodiments. The processor 108 of the system 100 may process the one or more acquired physiological characteristics.

In some embodiments, the processing can comprise comparing one or more physiological characteristics acquired when the information is indicative that the subject is in one posture with one or more corresponding physiological characteristics acquired when the information is indicative that the subject is in the same posture at another time. For example, the temperature of the at least one breast acquired when the subject is in one posture may be compared to the temperature of the at least one breast acquired when the subject is in the same posture at another time. In some examples, two or more corresponding physiological characteristics acquired when the subject is upright may be compared, two or more corresponding physiological characteristics acquired when the subject is lying on their side may be compared, or two or more corresponding physiological characteristics acquired when the subject is in any other same posture may be compared.

Alternatively or in addition, in some embodiments, one or more physiological characteristics acquired when the information is indicative that the subject is in one posture may be compared to one or more corresponding physiological characteristics acquired when the information is indicative that the subject is in another posture (i.e. a different posture), taking into account a transfer function of the one or more characteristics between the two postures. The transfer function may be acquired in a calibration phase in which the subject wears the garment 102 during a set time period (such as a whole day) and a relationship between corresponding physiological characteristics acquired while the subject is in different postures can be determined. In this way, acquired physiological characteristics can be calibrated to the posture of the subject. This can be useful where the subject only wears the garment 102 for a short period of time and only takes one, or a few, postures during that time period since one or more corresponding physiological characteristics acquired on days without the same postures occurring at different times can still be compared to each other.

Alternatively or in addition, in some embodiments, the processing may comprise comparing one or more physiological characteristics acquired when the information is indicative that the subject is in an active state with one or more corresponding physiological characteristic acquired when the information is indicative that the subject is in an inactive state (or in a static phase), taking into account a transfer function of the one or more characteristics between the active and inactive state. An active state is a state in which the subject is moving, or is moving more than a minimum threshold amount. An inactive state is a state in which the subject is not moving (i.e. is stationary, static or immobile) or is moving less than the minimum threshold amount.

Alternatively or in addition, in some embodiments, the processing may comprise comparing two or more physiological characteristics acquired during a defined motion with one more corresponding physiological characteristics acquired during the same defined motion. In other embodiments, the processing may comprise comparing two or more physiological characteristics acquired immediately following a defined motion with one more corresponding physiological characteristics acquired immediately following the same defined motion. In other embodiments, the processing may comprise comparing two or more physiological characteristics acquired during a defined motion with one more corresponding physiological characteristics acquired during the same defined motion and comparing two or more physiological characteristics acquired immediately following a defined motion with one more corresponding physiological characteristics acquired immediately following the same defined motion. A defined motion may, for example, be motion due to the movement of respiration or a stage of respiration (such as the exhale stage and the inhale stage), motion due to the movement of the subject changing position (such as moving from a sitting position to a standing position or vice versa), motion due to the movement of the subject changing posture (such as moving from a lying in a supine position to a sitting position or vice versa, or from lying on their side to lying supine), or any other defined motion. In this way, it is possible to compare the response of, and any changes in, the at least one breast to certain motions.

Alternatively or in addition, in some embodiments, the processing may comprise analysing one or more physiological characteristics acquired during a defined motion, immediately following a defined motion, or both during and immediately following a defined motion. For example, a response of the at least one breast to a defined motion may indicate whether the at least one breast is healthy. An example of a response may be the amount by which the at least one breast moves during a defined motion. In this example, a certain amount of movement may be indicative that the at least one breast is healthy and no movement (or limited amount movement) may be indicative of a potential issue with the at least one breast.

Alternatively or in addition, in some embodiments, the processing may comprise comparing one or more physiological characteristics acquired from at least one physiological characteristic sensor 104 positioned over a first breast with one or more corresponding physiological characteristics acquired from at least one physiological characteristic sensor 104 positioned over a second breast when the information is indicative of a change in the movement of the subject. In other words, in some embodiments, the processing may comprise comparing one or more acquired physiological characteristics of the first breast with one or more acquired physiological characteristics of the second breast.

In any of the embodiments described herein, a calibration phase may comprise comparing the one or more acquired physiological characteristics to a baseline for that physiological characteristic. In this way, the acquired physiological characteristics can be normalised such that comparisons can be made irrespective of changing external parameters (such as the time of day, the temperature, the location, or similar).

In any of the embodiments described herein, one or more physiological characteristics acquired when the information is indicative of the movement of the subject exceeding a threshold may be excluded from processing. In this way, it is possible to exclude physiological characteristics acquired when more than a threshold amount of movement occurs since movement can cause motion artefacts in the physiological characteristics. The exclusion of physiological characteristics may be performed during signal processing or data acquisition. For example, in some embodiments, one or more physiological characteristics may be excluded from processing by ceasing to acquire the one or more physiological characteristics from the at least one physiological characteristic sensor 104. Specifically, the one or more physiological characteristic sensors 104 may be turned off when movement is detected. This can aid in conserving power and improve data storage capacity.

In some embodiments, the method can then further comprise continuing to acquire and process the one or more physiological characteristics from the at least one physiological characteristic sensor when the information is indicative that the movement of the subject is equal to or below the threshold, or after a predetermined time period (for example, several minutes) has expired following the movement, or both. By waiting for a predetermined time period to expire following the movement, it can be ensured that the one or more physiological characteristics return to normal following a movement. For example, it can be ensured that the temperature of the at least one breast of the subject drops back to normal following the subject performing an activity (such as exercising).

Alternatively or in addition, in some embodiments, one or more physiological characteristics acquired before the information indicates that a predefined movement of the subject occurs may be excluded from processing. Thus, in some embodiments, a predefined movement of the subject may be required before processing of the acquired one or more physiological characteristics begins. The predefined movement may be characteristic of an activity that the subject is required to perform before physiological characteristics are processed to bring the at least one breast to a defined state.

In any of the embodiments described herein, the processing of the one or more acquired physiological characteristics may take into account a time in the life of the subject at which the one or more acquired physiological characteristics are acquired (such as the phase of the menstrual cycle, menopause, pregnancy, moment and frequency of lactation, or similar). In this way, more reliable results can be achieved from the processed information. Alternatively or in addition, in any of the embodiments described herein, the processing of the one or more acquired physiological characteristics may take into account an environment condition (such as room temperature, humidity, or any other environment condition, or combination thereof) at the time the one or more acquired physiological characteristics are acquired.

In any of the embodiments described herein, one or more user interfaces 112 may be controlled to output information resulting from the method. For example, in some embodiments, one or more user interfaces 112 may be controlled to output the one or more acquired physiological characteristics, or the information acquired from the one or more motion sensors 106, or the one or more acquired physiological characteristics and the information acquired from the one or more motion sensors 106. Alternatively or in addition, in some embodiments, one or more user interfaces 112 may be controlled to output the information indicative of any one or more of the abnormality and milk volume. In this way, it is possible to provide feedback on any differences or changes in the condition of the at least one breast.

The one or more acquired physiological characteristics may be processed for a variety of applications. For example, the one or more acquired physiological characteristics may be processed to categorise information on at least one breast, observe patterns in at least one breast, perform mapping (such as thermal gradient mapping, skin temperature mapping, skin feature mapping, or any other mapping) for one or more regions or the whole of at least one breast, to identify one or more abnormalities in at least one breast (such as glands, lumps, or similar), to identify a milk volume in at least one breast, to detect fullness or emptiness of at least one breast before or after breast-feeding or expressing milk, to determine which breast of a subject (specifically, a breast-feeding mother) can be used for breast feeding or milk extraction, to aid in preventing mastitis or breast inflammation, to detect complications with breast implants (such as wrinkling, asymmetry, rupture, leakage or similar), to support in predicting the pre-menstrual period, or any combination of these.

In some embodiments, processing the one or more acquired physiological characteristics for the at least one breast may comprise a comparison of the one or more acquired physiological characteristics to a baseline profile of the at least one breast. In these embodiments, possible changes as compared to the baseline profile can be rendered as an output. In some embodiments, processing the one or more acquired physiological characteristics for two breasts can comprise comparing the difference between the two breasts. In these embodiments, any variation in this difference can be rendered as an output.

There is therefore provided an improved system and method for breast monitoring. The system and method can be particularly useful in supporting a subject in the self-examination of breasts.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (100) for breast monitoring comprising:
a garment (102) adapted to at least partially cover at least one breast, the garment comprising at least one physiological characteristic sensor (104) configured to acquire one or more physiological characteristics of at least one breast of a subject wearing the garment (102); and
at least one motion sensor (106) configured to acquire information on the subject wearing the garment (102).

2. A system (100) as claimed in claim 1, wherein the information comprises any one or more of:
information indicative of a posture of the subject;
information indicative of an orientation of the subject;
information indicative of a position of the subject; and
information indicative of a movement of the subject.

3. A system (100) as claimed in any one of claims 1 or 2, wherein at least one motion sensor (106) is positioned over each breast of the subject.

4. A system as claimed in any one of claims 1 to 3, wherein the garment (102) comprises any one or more of:
one or more cups, each cup adapted to at least partially cover a breast of the subject, wherein one or more motion sensors (106) are positioned in at least one of the one or more cups or between the one or more cups, or in at least one of the one or more cups and between the one or more cups; and
one or more straps, wherein one or more motion sensors (106) are positioned in at least one of the one or more straps.

5. A system (100) as claimed in any one of the preceding claims, the system (100) further comprising:
a processor (108) configured to process the one or more acquired physiological characteristics based on the acquired information.

6. A system (100) as claimed in claim 5, wherein the garment (102) comprises the processor (108).

7. A method (200) for breast monitoring, the method comprising:
controlling (202) at least one physiological characteristic sensor (104) of a garment (102) adapted to at least partially cover at least one breast to acquire one or more physiological characteristics of at least one breast of a subject wearing the garment (102); and
controlling (204) at least one motion sensor (106) to acquire information on the subject wearing the garment (102).

8. A method (200) as claimed in claim 7, the method further comprising:
processing (206) the one or more acquired physiological characteristics based on the acquired information.

9. A method as claimed in claim 8, wherein processing the one or more physiological characteristics comprises any one or more of:
comparing one or more physiological characteristics acquired when the information is indicative that the subject is in one posture with one or more corresponding physiological characteristics acquired when the information is indicative that the subject is in the same posture at another time;
comparing one or more physiological characteristics acquired when the information is indicative that the subject is in one posture with one or more corresponding physiological characteristics acquired when the information is indicative that the subject is in another posture, taking into account a transfer function of the one or more characteristics between the two postures;
comparing one or more physiological characteristics acquired when the information is indicative that the subject is in an active state with one or more corresponding physiological characteristic acquired when the information is indicative that the subject is in an inactive state, taking into account a transfer function of the one or more characteristics between the active and inactive state;
comparing one or more physiological characteristics acquired during a defined motion, immediately following a defined motion, or both during and immediately following a defined motion with one more corresponding physiological characteristics acquired during the same defined motion, immediately following the same defined motion, or both during and immediately following the same defined motion;
analysing one or more physiological characteristics acquired during a defined motion, immediately following a defined motion, or both during and immediately following a defined motion; and
comparing one or more physiological characteristics acquired from at least one physiological characteristic sensor (104) positioned over a first breast with one or more corresponding physiological characteristics acquired from at least one physiological characteristic sensor (104) positioned over a second breast when the information is indicative of a change in the movement of the subject.

10. A method as claimed in any one of claims 8 or 9, wherein processing the one or more physiological characteristics comprises excluding from processing one or more physiological characteristics acquired when the information is indicative of the movement of the subject exceeding a threshold.

11. A method as claimed in claim 10, wherein excluding one or more physiological characteristics comprises:
ceasing to acquire the one or more physiological characteristics from the at least one physiological characteristic sensor (104).

12. A method as claimed in claim 11, wherein the method further comprises:
continuing to acquire and process the one or more physiological characteristics from the at least one physiological characteristic sensor (104) after a predetermined time period has expired following the movement or when the information is indicative that the movement of the subject is equal to or below the threshold.

13. A method as claimed in any one of claims 8 to 12, wherein the acquired one or more physiological characteristics are processed to identify any one or more of:
an abnormality in the at least one breast of the subject; and
a milk volume in the at least one breast of the subject.

14. A method as claimed in claim 13, the method further comprising:
controlling one or more user interfaces (112) to output information indicative of any one or more of the abnormality and milk volume.

15. A computer program product comprising a computer readable medium, the computer readable medium having computer readable code embodied therein, the computer readable code being configured such that, on execution by a suitable computer or processor, the computer or processor is caused to perform the method of any one of claims 7-14.
